# FASCICULE DE BREVET EUROPEEN

(11) **EP 0 853 660 B1**
(45) Date de publication et mention de la délivrance du brevet: **22.01.2003**
(21) Numéro de dépôt: 97933740.9
(22) Date de dépôt: 15.07.1997
(51) Int. Cl.: C12N 1/04, C12N 7/00, A61K 48/00

(54) **PROCEDE DE CONSERVATION DE VIRUS RECOMBINANTS INFECTIEUX, SUSPENSION AQUEUSE VIRALE ET UTILISATION COMME MEDICAMENT**
VERFAHREN ZUR KONSERVIERUNG VON INFEKTIÖSEN REKOMBINIERENDEN VIREN, VIRALE WÄSSRIGE SUSPENSION UND IHRE MEDIZINISCHE VERWENDUNG
METHOD FOR PRESERVING INFECTIOUS RECOMBINANT VIRUSES, AQUEOUS VIRAL SUSPENSION AND USE AS MEDICINE

(30) Priorité: 16.07.1996 FR 9608851
(43) Date de publication de la demande: 22.07.1998
(73) Titulaire: TRANSGENE S.A., 67000 Strasbourg (FR)
(72) Inventeur: SENE, Claude, F-67190 Mutzig (FR)
(74) Mandataire: Warcoin, Jacques
(86) Numéro de dépôt international: FR9701308
(87) Numéro de publication internationale: WO98002522

(56) Documents cités:
- WO-A-95/10601
- US-A- 5 545 555

## Description

L'invention concerne un procédé de conservation de virus recombinants infectieux, une suspension aqueuse virale, et son utilisation comme médicament.

Les virus vivants ont de nombreuses applications, en particulier comme vaccins. Ce sont des particules qui possèdent un génome sous forme d'ADN ou d'ARN contenant les informations utiles à leur replication, mais qui ont besoin d'infecter une cellule hôte pour réaliser la synthèse des protéines qui leur sont nécessaires.

Par ailleurs, la possibilité d'intégrer dans un génome de virus du matériel génétique étranger a permis de créer des virus dits recombinants portant un gène d'intérêt thérapeutique utilisés pour transférer ce gène dans des cellules spécifiques de patients déficients. C'est le principe de la thérapie génique.

La possibilité de traiter les maladies humaines par thérapie génique est passée en quelques années du stade des considérations théoriques à celui des applications cliniques. La grande majorité des protocoles décrits jusqu'à présent met en oeuvre des vecteurs viraux pour transférer et exprimer le gène thérapeutique dans les cellules à traiter.

A ce jour, les vecteurs rétroviraux sont parmi les plus utilisés du fait de la simplicité de leur génome, bien qu'ils présentent une capacité de clonage assez restreinte.

Les adénovirus, quant à eux, offrent plusieurs avantages qui en font des vecteurs de choix pour une grande variété d'applications. En effet, ils infectent de nombreux types cellulaires, sont non-intégratifs, peu pathogènes et peuvent se repliquer dans les cellules en division ou quiescentes. A titre indicatif, leur génome est constitué d'une molécule d'ADN linéaire et bicaténaire d'environ 36 kb portant plus d'une trentaine de gènes, à la fois des gènes précoces nécessaires à la replication virale (E1 à E4 ; E pour early, signifiant précoce en anglais) et des gènes tardifs de structure (L1-L5 ; L pour late, signifiant tardif en anglais).

Les vecteurs adénoviraux recombinants utilisés à des fins de thérapie génique sont déficients pour la replication afin d'éviter leur dissémination dans l'environnement et l'organisme hôte. D'une manière générale, ils sont dépourvus de la majorité de la région E1 et certains d'inflammation liés à l'expression des gènes viraux restants. Ils ne peuvent être propagés que par transcomplémentation des fonctions adénovirales pour lesquelles ils sont déficients. A l'heure actuelle, on utilise essentiellement la lignée de complémentation 293 (Graham et al., 1977, J. Gen. Virol. 36, 59-72) ou des lignées en dérivant (Yeh et al., 1996, J. Virol. 70, 559-565 ; Wang et al., 1995, Gene Therapy 2, 775-783 ; Krougliak et Graham, 1995, Human Gene Therapy 6, 1575-1586).

Les adénovirus sont notamment utilisés dans le cas du traitement de la mucoviscidose par thérapie génique (Pavirani et al., 1996, médecine /sciences 12, 25-33).

Les virus recombinants ne sont cependant utilisables que si leur viabilité et leur infectuosité ont été convenablement préservés pendant toute la période de stockage.

Traditionnellement, les adénovirus purifiés sont conservés dans une solution saline contenant du glycérol de 10 à 30 % (Graham et al., 1991, Methods in Molecular Biology, vol. 7, chap. 11, p. 109-127 ; Ed Murrey, The Human Press Inc. ; Precious et Russel, Virology, a Practical Approach, 1985, chap. 9, p. 193-205 ; ed : BW Mahy, IRL Press, Washington DC ; Kanegae et al., Jpn. J. Med. Sci. Biol., 47, 157-166, 1994 et Green et al., Methods in enzymology, vol. LVIII, p. 425-435). Cependant, le glycérol présente l'inconvénient d'être irritant pour l'épithélium pulmonaire, ce qui peut être délicat dans le cadre d'une administration intratrachéale et intrapulmonaire (par exemple pour le traitement de la mucoviscidose ou de cancers des voies pulmonaires). De plus, si cette solution permet la conservation des adénovirus sous forme congelée, elle ne permet pas de maintenir leur activité à + 4°C au-delà d'une semaine.

L'addition de sucrose à faible concentration (1 à 5 %) dans une solution saline a également été décrite (Precious et al., voir ci-dessus ; Huyghe et al., Human Gene Therapy 6:1403-1416, Novembre 1995, et Hehir, Process Development and Production Issues for Viral Vectors & Vaccines, The Williamsburg Bio Processing Conference, 2nd annual meeting, 6-9 Novembre 1995) permettant une stabilité des adénovirus à long terme sous forme congelée, mais seulement à court terme à + 4°C (Hehir, voir ci-dessus).

La conservation des virus sous forme congelée posant des problèmes de stockage et de transport, il a également été envisagé de préserver les virus et les vaccins viraux sous une forme lyophilisée. Cependant, cette technique présente l'inconvénient d'entraîner souvent une perte de l'activité virale. Pour pallier cela, l'addition d'excipients comme les sucres (sucrose, glucose, tréhalose), permet de maintenir l'activité virale sous forme lyophilisée (WO 95/10601 - Viagene et EP-0 357 709 - Quadrant). L'emploi du lactose ou du saccharose à faible concentration (2,5 - 5 %) pour la préservation du virus vivant atténué sous forme lyophilisée a également été préconisée (JP-88 555465 - Kitasako Inst.).

Aucune des solutions proposées jusqu'à présent n'a permis de maintenir l'activité des adénovirus à des niveaux satisfaisants pendant plus de 6 mois, tout en évitant les problèmes secondaires tels que les problèmes d'irritation.

La présente invention remédie aux inconvénients de l'art antérieur. Elle a pour objet un procédé de conservation à long terme de virus recombinants infectieux tant sous forme liquide que sous forme congelée, dans lequel les virus recombinants sont conservés dans une solution aqueuse comprenant du saccharose à haute concentration.

En effet, si l'utilisation du saccharose à haute concentration est connue depuis longtemps pour conserver les protéines ou autres produits biologiques (Timasheef et al., In Protein Structure, a Practical Approach, 1989, Ed Creighton, chap. 14, p. 331-345, IRL Press, Oxford, et Doebbler, Cryobiology, vol. 3, N° 1, 1966) ou pour la cryopréservation des cellules vivantes dans l'azote liquide (Grout et al., Tibtech, Octobre 1990, vol. 8, p. 293-297), elle n'a jamais été proposée pour la conservation des virus.

Or, les résultats obtenus par la mise en oeuvre du procédé selon l'invention ont démontré un effet cryoprotecteur du saccharose à différentes températures de stockage (- 80 °C, - 40°C, - 20°C et + 4°C) et ceci d'autant plus que la concentration en saccharose est élevée.

Avantageusement, les virus recombinants infectieux concernés par la présente invention sont des poxvirus, des adénovirus, des virus associés aux adénovirus et des rétrovirus.

Dans le cadre de l'invention, les virus sont conservés dans une solution aqueuse comprenant du saccharose à haute concentration, c'est-à-dire à une concentration supérieure à 0,75 M, de préférence comprise entre 0,75 M et 1,5 M, plus préférentiellement encore à une concentration égale à 1 M.

Les virus recombinants infectieux gagnent en stabilité quand la solution aqueuse utilisée présente un pH basique compris entre 8 et 9, et de préférence égal à 8,5.

Ainsi, la solution aqueuse mise en oeuvre dans le cadre de la présente invention peut être une solution tampon choisie parmi le tampon Tris, les solutions triéthanolamine, diéthanolamine, borate/HCl, Glycine /NaOH, EPPS [acide N-(2-hydroxyéthyl) pipérazine-N'-(3-propanesulfonique)], Bicine, TAPS [acide N-tris (hydroxyméthyl) méthyl-3-aminopropane sulfonique] et tricine.

Avantageusement, il est possible de stabiliser davantage la capside ou l'enveloppe virale des virus conservés selon l'invention en ajoutant à la solution aqueuse utilisée, au moins un sel d'un cation divalent choisi parmi le MgCl₂, le CaCl₂ et le MnCl₂, le MgCl₂ étant préféré.

Dans le cadre de la présente invention, le sel de cation divalent est utilisé à une concentration comprise entre 0,1 et 5 mM, de préférence entre 0,5 et 2 mM et encore plus préférentiellement, de l'ordre de 1 mM.

Selon un mode de réalisation avantageux de procédé conforme à l'invention, les virus sont conservés dans une solution tampon comprenant un tampon Tris HCl 10 mM, MgCl₂ 1 mM, saccharose 1 M, pH 8,5.

On peut encore améliorer la conservation des virus en utilisant au moins un composé stabilisant choisi parmi les sels, de préférence monovalents tels que le NaCl ou le KCl qui procurent une force ionique à la solution, des acides aminés tels que Gly, Leu, Lys, Arg, Asp, Val, Glu et les composés agissant sur la tension de surface tels que le Tween 80 ou le Triton X-100, ces derniers pouvant être utilisés seuls ou en présence de sels.

Avantageusement, à titre de composé stabilisant, le NaCl est utilisé à une concentration comprise entre 0,05 et 1 M, de préférence entre 0,1 et 0,5 M, plus préférentiellement encore entre 0,1 et 0,3 M, la concentration considérée comme optimale étant de 0,15 M, et le Tween 80 est utilisé à une concentration comprise entre 0,001 et 0,5 % en poids par rapport à la solution totale (soit entre 10 mg/l et 5 g/l), de préférence entre 0,002 et 0,2 % en poids et plus préférentiellement encore de l'ordre de 0,005 % en poids.

Selon un mode de réalisation préféré, le procédé selon l'invention met en oeuvre une solution aqueuse de pH environ 8,5 comprenant du Tris-HCl 10 mM, du MgCl₂ 1mM, du NaCl 0,9 % (ou 150 mM) de Tween 80 50 mg/l (0,05 %) et du saccharose 1 M.

De plus, les virus recombinants infectieux conservés selon le procédé conforme à l'invention, peuvent être soumis à une lyophilisation.

L'invention a également pour objet une suspension aqueuse de virus recombinants infectieux dans une solution aqueuse de saccharose à haute concentration telle que précédemment décrite.

Avantageusement, la suspension aqueuse conforme à l'invention comprend de 106 à 10¹³ pfu/ml de virus recombinants infectieux.

La présente invention concerne également une composition pharmaceutique comprenant une suspension aqueuse de virus recombinants infectieux telle que ci-dessus décrite ou obtenue par la mise en oeuvre du procédé de conservation conforme à l'invention, en association avec un véhicule acceptable d'un point de vue pharmaceutique. Elle peut être administrée par voie systémique, en particulier par voie sous-cutanée, intraveineuse, intracardiaque, intramusculaire, intrapéritonéale, intragastrique, intratumorale, intrapulmonaire, intranasale ou intratrachéale. L'administration peut avoir lieu en dose unique ou repétée une ou plusieurs fois après un certain délai d'intervalle. La formulation peut également inclure d'autres composés tels qu'un adjuvant ou un excipient acceptable d'un point de vue pharmaceutique. Une composition selon l'invention est, en particulier, destinée au traitement préventif ou curatif de maladies telles que les maladies génétiques (hémophilie, mucoviscidose, diabète, myopathie de Duchenne et de Becker, ...), les cancers, les maladies virales (hépatites, SIDA, ...) et les maladies récurrentes (infections provoquées par le virus de l'herpès, le papilloma humain, ...).

Enfin, la présente invention est relative à l'usage thérapeutique ou prophylactique d'une suspension aqueuse de virus recombinants infectieux telle que ci-dessus décrite ou obtenue par la mise en oeuvre du procédé de conservation conforme à l'invention, pour la préparation d'un médicament destiné au traitement du corps humain ou animal par thérapie génique. La suspension aqueuse peut être administrée directement in vivo (par exemple par injection intraveineuse, intramusculaire, dans une tumeur accessible, dans les poumons par aérosol, ...). On peut également adopter l'approche ex vivo qui consiste à prélever des cellules du patient (cellules souches de la moëlle osseuse, lymphocytes du sang périphérique, cellules musculaires, ...), de les infecter par la suspension aqueuse de l'invention selon les techniques de l'art et de les réadministrer au patient.

La figure 1 illustre l'influence du pH de la solution de saccharose sur la stabilité virale.

La figure 2 illustre l'influence de l'addition à la solution de saccharose de Tween 80 et de NaCl. L'unité de l'ordonnée est exprimée en pfu/ml.

D'autres caractéristiques de l'invention apparaîtront à la lumière des exemples suivants.

### MATÉRIELS ET MÉTHODES

Les exemples qui suivent mettent en oeuvre un vecteur adénoviral recombinant exprimant soit le gène marqueur *LacZ* codant pour la β-galactosidase d'*E*. *coli* ou le gène thérapeutique CF codant pour la protéine CFTR (Cystic Fibrosis Transmembrane conductance Regulator) déficiente chez les patients atteints de mucoviscidose. A titre indicatif, le vecteur est obtenu à partir du génome de l'adénovirus de type 5 (Ad5) délété des régions E1 et E3, et comprend, intégrée à la place de la région E1, une cassette pour l'expression du gène marqueur ou thérapeutique (Stratford-Perricaudet et al., 1992, J. Clin. Invest. 90, 626-630; Rosenfeld et al., 1992, Cell. 68, 143-155). Il peut être propagé dans la lignée 293 (Graham et al., 1977, J. Gen. Virol. 36, 59-72) complémentant la fonction E1 essentielle à la réplication virale. A titre indicatif, la lignée 293 dérive de rein embryonnaire humain et résulte de l'intégration dans ses chromosomes de l'extrémité 5' du génome de l'Ad5 (11 %). Les cellules 293 sont disponibles à l'ATCC (CRL1573) et sont cultivées selon les conditions préconisées par le fournisseur ou dans la littérature.

Un stock viral primaire est constitué de manière conventionnelle dans les cellules 293 transfectées par le vecteur adénoviral décrit ci-dessus. On vérifie la production de particules virales infectieuses recueillies après lyse des cellules par des cycles consécutifs de congélation-décongélation, le titre de la préparation virale par la méthode agar (Graham et Prevec, 1991, Methods in Molecular Biology, vol. 7, p 109-128 ; Ed: E. J. Murey, The Human Press Inc.) et l'expression du gène marqueur par coloration au Xgal (4-chloro-5-bromo-3-indolyl-β-galactosidase) selon la procédure de Sanes et al. (1986, EMBO J. 5, 3133-3142) ou du gène CF par Western blot à l'aide d'anticorps spécifiques (Dalemans et al., 1992, Experimental Cell Research 201, 235-240). La préparation virale peut être purifiée et concentrée par gradient de densité préalablement à son utilisation.

### EXEMPLE 1 :

### Influence du pH sur la stabilité du virus

Une suspension virale est préparée de la manière suivante.

Les cellules 293 sont cultivées en CellCube (Costar) dans un milieu GMEM supplémenté avec 7 % de sérum de veau foetal (FCS). Lorsqu'elles atteignent la confluence, elles sont infectées par une aliquote du stock primaire du vecteur adénoviral exprimant le gène CF à une m.o.i. (multiplicité d'infection) de 2. Trente heures après l'infection, les cellules qui sont fragilisées, sont détachées par agitation mécanique ou à l'aide d'un agent chimique et récoltées par centrifugation à basse vitesse (3 500 rpm (révolutions par minute) pendant 8 minutes). Elles sont lysées et les particules virales libérées par 3 séries de congélation-décongélation et les débris cellulaires éliminés par centrifugation (3 500 rpm pendant 8 minutes). Le virus est purifié à partir du surnageant par deux ultracentrifugations sur chlorure de césium (CsCl), la première sur coussins de CsCI de densité d = 1,25 et d = 1,40 respectivement (141 000 g pendant 2 heures) et la seconde sur un gradient autoformé à partir d'une solution de CsCI de densité d = 1,34 (231 000 g pendant 18 heures).

La bande de virus est récupérée, son titre déterminé (2 x 10¹¹ pfu) et la préparation est divisée en 4 lots qui sont soumis à une dialyse à 4°C contre 4 fois 250 ml de tampon Tris 10 mM, MgCl₂ 1 mM, glycérol 10 %, de pH croissant : pH 7,4, pH 8, pH 8,5 et pH 9 respectivement. La stabilité virale dans les différents tampons de formulation est étudiée en parallèle (étude de stabilité accélérée). Pour ce faire, chaque lot est conditionné en cryotubes de 1 ml contenant chacun 100 µl de suspension. Les échantillons sont incubés à 37°C et prélevés à tₒ et après 4, 24 et 72 h d'incubation. Ils sont conservés à - 20°C jusqu'au titrage. Le titre viral est déterminé par la méthode agar par réinfection de cellules 293 par différentes dilutions de l'échantillon à tester. Les résultats sont donnés en pfu (unité formant des plages)/ml.

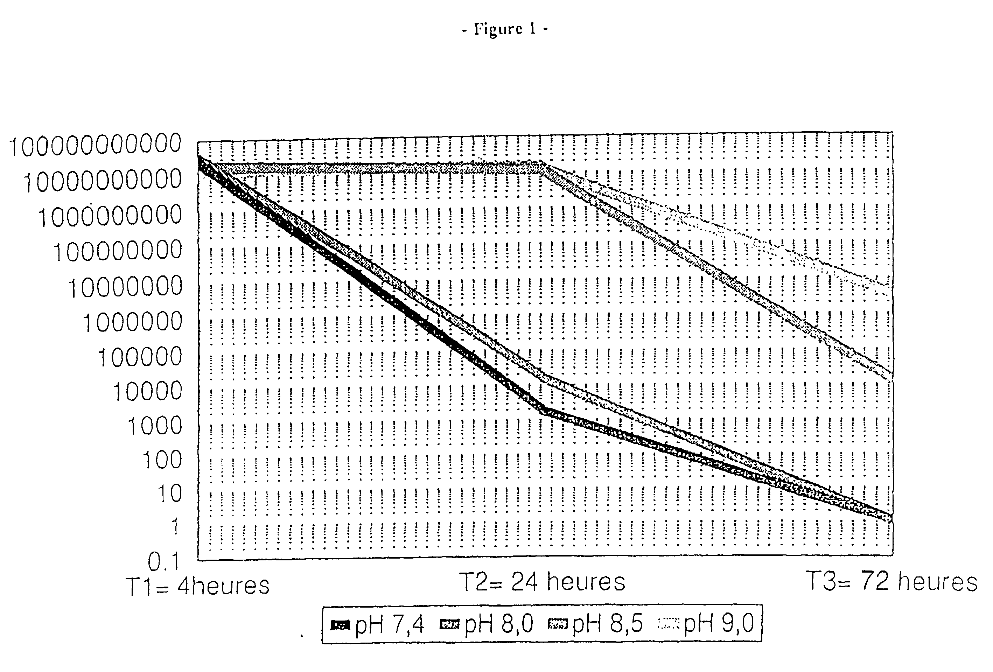

Les résultats présentés sur la Figure 1 montrent que la formulation à pH basique préserve l'activité infectieuse des adénovirus. En effet, le titre des préparations formulées dans les tampons à pH 8,5 et 9 sont stables pendant 24 heures à 37°C puis diminuent progressivement au cours du temps. En revanche, les virus placés dans un tampon à pH 7,4 et 8 perdent leur pouvoir infectieux dès le début de l'incubation à 37°C. Après 24 heures, les titres sont déjà très bas (10³ à 10⁴ pfu/ml contre de l'ordre de 10¹⁰ au départ) et les virus ne sont pratiquement plus infectieux au bout de 72 heures.

### EXEMPLE 2 :

### Influence de la concentration en Saccharose sur la stabilité du virus

Une suspension virale est préparée comme décrit à l'exemple 1 avec les quelques modifications suivantes :
- les cellules sont infectées par le vecteur adénoviral exprimant le gène *LacZ* ;
- les cellules infectées sont lysées de manière mécanique (homogéneiseur Silverson ; référence L4R) ;
- les ultracentrifugations en gradient de CsCI sont réalisées à l'aide de rotors à angle fixe (235 000 g pendant 2 h pour la première et 435 000 g pendant 18 h pour la seconde) ;
- la dialyse est remplacée par une étape de chromatographie de filtration sur gel à l'aide de la matrice Trisacryl GF05 LS (Biosepra, référence 259161) permettant un dessalage de la solution par élimination du CsCl ;
- les virus sont formulés dans une solution de Tris-HCl 10 mM, MgCl₂ 1 mM et saccharose 1 M d'un pH de 8,5 avant d'étre répartis en 5 lots par dilution au 1/20 dans les tampons indiqués ci-après et préalablement filtrés sur membrane de porosité 0,22 µm
   Lot 1) Tris-HCl 10 mM MgCl₂ 1 mM Saccharose 1 M pH 8,5
   Lot 2) Tris-HCl 10 mM MgCl₂ 1 mM Saccharose 0,75 M pH 8,5
   Lot 3) Tris-HCl 10 mM MgCl₂ 1 mM Saccharose 0,5 M pH 8,5
   Lot 4) Tris-HCl 10 mM MgCl₂ 1 mM Saccharose 0,25 M pH 8,5
   Lot 5) Tris-HCl 10 mM MgCl₂ 1 mM Saccharose 0 M pH 8,5

Chacun des lots titre au départ environ 10¹⁰ pfu/ml. La stabilité des virus est mesurée en condition accélérée (37°C) sur des aliquotes prélevées régulièrement.

Les résultats sont présentés dans le tableau 1 suivant :

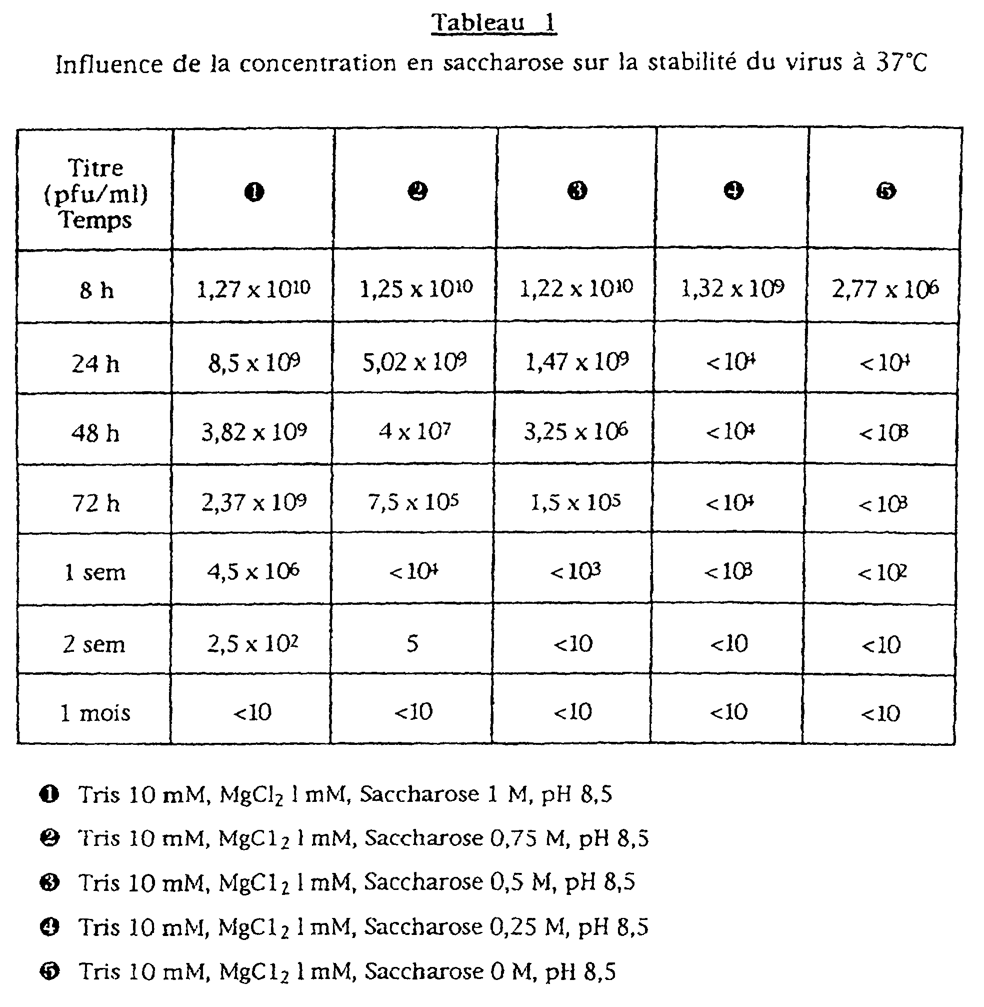

De cette étude, il ressort que plus la concentration en saccharose est élevée, plus l'activité virale est préservée (lot 1 plus stable que le lot 2 lui-même plus stable que le lot 3, etc.). En absence de saccharose (lot 5), le pouvoir infectieux chute très rapidement (diminution d'un facteur 5000 dès 8 heures d'incubation). En présence de 0,25 M (lot 4), le titre diminue rapidement mais à un moindre degré (diminution d'un facteur 10 après 8 heures à 37°C). En augmentant encore les concentrations en saccharose (lots 1, 2 et 3), le titre se maintient pendant plus de 8 heures puis décroît progressivement au fur et à mesure de l'incubation. La décroissance est cependant minime lorsque la concentration en saccharose atteint 1M (lot 1).

### EXEMPLE 3 :

### Etude de la stabilité à long terme dans le tampon de formulation Tris-HCl 10 mM, MgCl₂ 1 mM, saccharose 1 M, pH 8,5

L'étude est réalisée à partir de la suspension virale obtenue à l'exemple 2, dont on étudie la stabilité en conditions long terme à + 4°C et - 20°C. Le titre viral est suivi au cours du temps par titrage agar et les résultats indiqués dans le tableau 2 ci-après montrent une stabilité des préparations virales formulées en présence de saccharose 1 M et à pH 8,5 pendant au moins 6 mois.

**Tableau 2**

| Etude de stabilité à + 4°C et - 20°C d'une préparation virale formulée en saccharose 1M. | | |
|---|---|---|
| Titres viraux en pfu/ml : | | |
| Temps | + 4°C | - 20°C |
| t₀ | 4,8 x 10⁹ | |
| 1 mois | 1 x 10¹⁰ | 9,75 x 10⁹ |
| 2 mois | 9,25 x 10⁹ | 1,38 x 10¹⁰ |
| 3 mois | 1,32 x 10¹⁰ | 1,05 x 10¹⁰ |
| 6 mois | 1,1 x 10¹⁰ | 1,0 x 10¹⁰ |
| 1 an | 3,7 x 10⁹ | 5,0 x 10⁹ |

Le tampon de formulation en saccharose 1 M a également été comparé au tampon conventionnel (Tris-HCl 10 mM, MgCl₂ 1 mM, glycérol 10 %, pH 7,4). Cette étude a été menée à + 4°C sur une suspension virale diluée à un titre final d'environ 10¹⁰ pfu/ml dans les 2 types de tampon. Les résultats sont présentés dans le tableau 3 ci-après.

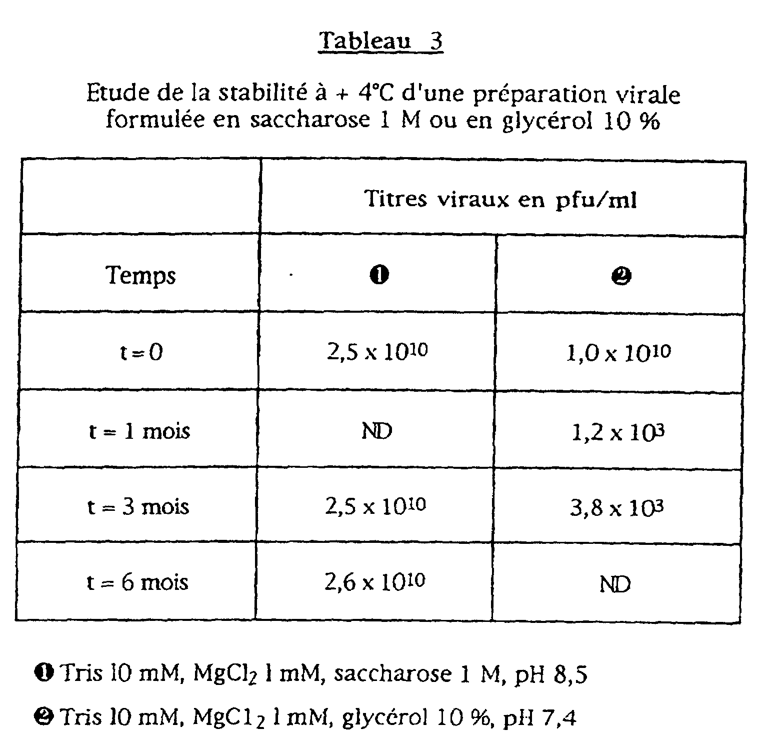

Le titre viral est stable pendant plus de 6 mois à + 4°C lorsque le tampon de formulation comprend du saccharose 1 M et à un pH légèrement basique alors qu'il décroit dès le premier mois lorsque les virus sont placés à pH neutre et en présence de glycérol 10 %.

### EXEMPLE 4:

### Optimisation du tampon de formulation

La suspension virale obtenue à l'exemple 2 est répartie en 2 lots dilués au 1/20 dans le tampon de formulation utilisé pour le lot 1) non supplémenté ou en présence de Tween 80 50 mg/l (0,005 %) et NaCl 150 mM. La stabilité est analysée à 37°C et à 4°C.

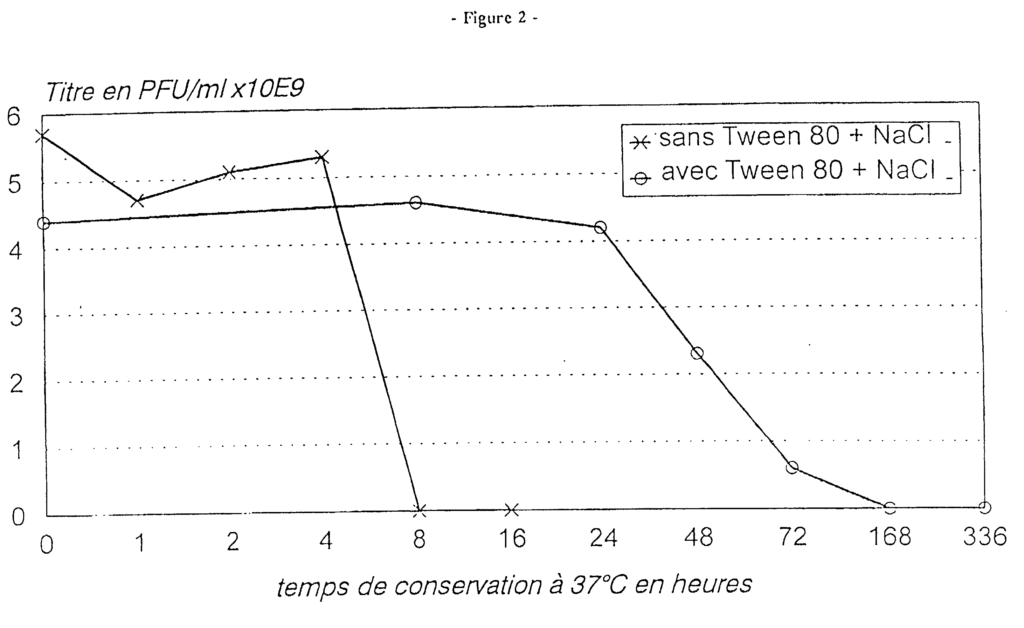

Les résultats de stabilité accélérée (Figure 2) montrent que l'ajout d'agents conservateurs tels que le Tween 80 et le sel améliore encore la stabilité des virus formulés en tampon Tris-HCl 10 mM, MgCl₂ 1 mM, saccharose 1 M, pH 8,5. L'activité infectieuse se maintient pendant 24 h à 37°C en leur présence au lieu de 8 h en leur absence.

Par ailleurs, la présence des 2 agents conservateurs ne nuit pas à la stabilité de la préparation virale à + 4°C puisque le titre s'avère stable pendant plus de 6 mois.

### EXEMPLE 5 :

### Stabilité dans le tampon de formulation Tris-HCl 10 mM, MgCl₂ 1mM, NaCI 0.9 %, Tween 80 50 mg 1l et saccharose 1 M, pH 8,5.

Une suspension virale comme décrit à l'exemple 2 formulée dans une solution de Tris-HCl 10 mM, MgCl₂ 1mM et saccharose 1 M est diluée au 1/20 dans le tampon de formulation suivant :

| | |
|---|---|
| Tris-HCl | 10 mM |
| MgCl₂ | 1 mM |
| NaCl | 0,9% (150 mM) |
| Tween 80 | 50 mg/l |
| Saccharose | 1 M |
| pH 8,5 | |

L'échantillon est conditionné en cryotubes de 1 ml, contenant chacun 100 µl de suspension virale. Les cryotubes sont conservés à + 4°C et les titres viraux déterminés à t₀ et régulièrement dans le temps pendant 1 an. Les échantillons sont conservés à - 20°C jusqu'au titrage. Les résultats sont présentés dans le tableau 4 suivant et montrent une stabilité des virus pendant au moins 1 an.

**Tableau 4**

| **Temps de conservation à 4°C** | **Titre en pfu/ml** |
|---|---|
| t₀ | 5,5 x 10⁹ |
| 2 semaines | 5,6 x 10⁹ |
| 1 mois | 23 x 10⁹ |
| 2 mois | 4 x 10⁹ |
| 3 mois | 5,6 x 10⁹ |
| 6 mois | 4,5 x 10⁹ |
| 1 an | 2,5 x 10⁹ |

## Revendications

1. Procédé de conservation de virus recombinants infectieux sous forme congelée ou liquide, dans lequel les virus sont conservés dans une solution aqueuse comprenant du saccharose à une concentration supérieure à 0,75 M, de préférence comprise entre 0,75 M et 1,5 M, plus préférentiellement à une concentration égale à 1 M, le pH de ladite solution aqueuse étant compris entre 8 et 9, et de préférence étant égal à 8,5.

2. Procédé de conservation de virus recombinants infectieux selon la revendication 1, dans lequel les virus sont des adénovirus ou des rétrovirus.

3. Procédé de conservation de virus recombinants infectieux selon l'une des revendications 1 à 2, dans lequel la solution aqueuse est une solution tampon.

4. Procédé de conservation de virus recombinants infectieux selon la revendication 3, dans lequel la solution tampon est choisie parmi le tampon Tris-HCl, les solutions triéthanolamine, diéthanolamine, borate / HCl, Glycine / NaOH, EPPS [acide N-(2-hydroxyéthyl) pipérazine-N'-(3-propane sulfonique)], Bicine, TAPS [acide N-tris (hydroxyméthyl) méthyl-3-aminopropane sulfonique] et tricine, le tampon Tris-HCl étant préféré.

5. Procédé de conservation de virus recombinants infectieux selon l'une des revendications 1 à 4, dans lequel la solution aqueuse comprend en outre au moins un sel d'un cation divalent choisi parmi MgCl₂, CaCl₂ et MnCl₂, MgCl₂ étant préféré.

6. Procédé de conservation de virus recombinants infectieux selon la revendication 5, dans lequel le sel de cation divalent est présent dans la solution aqueuse à une concentration comprise entre 0,1 et 5 mM, de préférence entre 0,5 et 2 mM et encore plus préférentiellement, de l'ordre de 1 mM.

7. Procédé de conservation de virus recombinants infectieux selon l'une des revendications 1 à 6, dans lequel la solution aqueuse comprend un tampon Tris-HCl 10 mM, MgCl₂ 1 mM, saccharose 1 M, pH 8,5.

8. Procédé de conservation de virus recombinants infectieux selon l'une des revendications 1 à 6, dans lequel la solution aqueuse comprend au moins un composé stabilisant choisi parmi les'sels, de préférence monovalents, les acides aminés et les tensio-actifs.

9. Procédé de conservation de virus recombinants infectieux selon la revendication 8, dans lequel le sel monovalent est choisi parmi NaCl et KCl, le NaCl étant préféré.

10. Procédé de conservation de virus recombinants infectieux selon la revendication 9, dans lequel le sel monovalent est présent dans la solution aqueuse à une concentration comprise entre 0,05 et 1 M, de préférence 0,1 et 0,5 M, plus préférentiellement encore entre 0,1 et 0,3 M.

11. Procédé de conservation de virus recombinants infectieux selon la revendication 8, dans lequel le tensio-actif est le Tween 80 ou le Triton X-100.

12. Procédé de conservation de virus recombinants infectieux selon la revendication 11, dans lequel le Tween 80 est présent dans la solution aqueuse à une concentration comprise entre 0,001 et 0,5 %, de préférence entre 0,002 et 0,2 %, et plus préférentiellement encore de l'ordre de 0,005 % en poids par rapport à la solution totale.

13. Procédé de conservation de virus recombinants infectieux selon la revendication 12, dans lequel la solution aqueuse comprend un tampon Tris-HCI 10mM, MgCl₂ 1mM, NaCl 150 mM, Tween 80 0,05 % et saccharose 1 M, pH environ 8,5.

14. Procédé de conservation de virus recombinants infectieux selon l'une des revendications 1 à 13, dans lequel la température de conservation est inférieure ou égale à + 4°C.

15. Procédé de conservation de virus recombinants infectieux selon l'une des revendications 1 à 14, dans lequel les virus en solution sont ensuite soumis à une lyophilisation.

16. Suspension aqueuse de virus recombinants infectieux comprenant une solution aqueuse de saccharose à une concentration supérieure à 0,75 M, de préférence comprise entre 0,75 M et 1,5 M, plus préférentiellement encore égale à 1 M, ladite solution aqueuse présentant un pH compris entre 8 et 9, de preférence un pH égal à 8,5.

17. Suspension aqueuse de virus recombinants infectieux selon la revendication 16, **caractérisée en ce que** les virus sont des adénovirus ou des rétrovirus.

18. Suspension aqueuse de virus recombinants infectieux selon l'une des revendications 16 et 17, dans laquelle la solution aqueuse est une solution tampon.

19. Suspension aqueuse de virus recombinants infectieux selon la revendication 18, dans laquelle la solution tampon est choisie parmi le tampon Tris-HCl, les solutions triéthanolamine, diéthanolamine, borate / HCl, Glycine / NaOH, EPPS [acide N-(2-hydroxyéthyl) pipérazine-N'-(3-propanesulfonique)], Bicine, TAPS [acide N-tris (hydroxyméthyl) méthyl-3-aminopropane sulfonique] et tricine, le tampon Tris-HCl étant préféré.

20. Suspension aqueuse de virus recombinants infectieux selon l'une des revendications 16 à 19, dans laquelle la solution aqueuse comprend en outre au moins un sel d'un cation divalent choisi parmi MgCl₂, CaCl₂ et MnCl₂, MgCl₂ étant préféré.

21. Suspension aqueuse de virus recombinants infectieux selon la revendication 20, dans laquelle le sel de cation divalent est présent dans la solution aqueuse à une concentration comprise entre 0,1 et 5 mM, de préférence entre 0,5 et 2 mM et encore plus préférentiellement, de l'ordre de 1 mM.

22. Suspension aqueuse de virus recombinants infectieux selon l'une des revendications 16 à 21, dans laquelle la solution aqueuse comprend un tampon Tris HCl 10 mM, MgCl₂ 1 mM, saccharose 1 M, pH 8,5.

23. Suspension aqueuse de virus recombinants infectieux selon l'une des revendications 16 à 21, dans laquelle la solution aqueuse comprend au moins un composé stabilisant choisi parmi les sels, de préférence monovalents, les acides aminés et les tensio-actifs.

24. Suspension aqueuse de virus recombinants infectieux selon la revendication 23, dans laquelle le sel monovalent est choisi parmi NaCl et KCl, le NaCl étant préféré.

25. Suspension aqueuse de virus recombinants infectieux selon la revendication 24, dans laquelle le sel monovalent est présent dans la solution aqueuse à une concentration comprise entre 0,05 et 1 M, de préférence 0,1 et 0,5 M, plus préférentiellement encore entre 0,1 et 0,3 M.

26. Suspension aqueuse de virus recombinants infectieux selon la revendication 23, dans laquelle le tensio-actif est le Tween 80 ou le Triton X-100.

27. Suspension aqueuse de virus recombinants infectieux selon la revendication 26, dans laquelle le Tween 80 est présent dans la solution aqueuse à une concentration comprise entre 0.001 et 0,5 %, de préférence entre 0,002 et 0,2 %, et plus préférentiellement encore de l'ordre de 0,005 % en poids par rapport à la solution totale.

28. Suspension aqueuse de virus recombinants infectieux selon l'une des revendications 16 à 27, comprenant de 10⁶ à 10¹³ pfu/ml de virus recombinants infectieux.

29. Suspension aqueuse selon la revendication 27, comprenant un tampon Tris-HCl 10 mM; MgCl₂ 1mM, NaCl 150 mM, Tween 80 0,05 % et du saccharose 1M, pH environ 8,5.

30. Composition pharmaceutique comprenant une suspension aqueuse de virus recombinants infectieux selon l'une des revendications 16 à 28, ou obtenue par la mise en oeuvre d'un procédé de conservation des virus recombinants infectieux selon les revendications 1 à 15, en association avec un véhicule acceptable d'un point de vue pharmaceutique.

31. Usage d'une suspension aqueuse de virus recombinants infectieux selon l'une des revendications 16 à 28, ou obtenue par la mise en oeuvre d'un procédé de conservation des virus recombinants infectieux selon les revendications 1 à 15, pour la préparation d'un médicament destiné au traitement ou à la prophylaxie des maladies génétiques, des cancers, des maladies virales et des maladies récurrentes.

32. Usage selon la revendication 31, pour la préparation d'un médicament destiné au traitement ou à la prophylaxie de l'hémophilie, la mucovisidose, le diabète, la myopathie de Duchenne et de Becker.

33. Usage selon la revendication 31, pour la préparation d'un médicament destiné au traitement ou à la prophylaxie des hépatites ou du SIDA.

34. Usage selon la revendication 31, pour la préparation d'un médicament destiné au traitement ou à la prophylaxie des infections provoquées par le virus de l'herpès ou le papilloma humain.

## Patentansprüche

1. Verfahren zur Konservierung von infektiösen rekombinanten Viren in gefrorener oder flüssiger Form, in dem die Viren in einer wäßrigen Lösung konserviert werden, welche Saccharose bei einer Konzentration über 0,75 M, vorzugsweise von 0,75 M bis 1,5 M, bevorzugter bei einer Konzentration gleich 1 M umfasst, wobei der pH der wäßrigen Lösung 8 bis 9 beträgt und vorzugsweise gleich 8,5 ist.

2. Verfahren zur Konservierung von infektiösen rekombinanten Viren nach Anspruch 1, in dem die Viren Adenoviren oder Retroviren sind.

3. Verfahren zur Konservierung von infektiösen rekombinanten Viren nach einem der Ansprüche 1 bis 2, in dem die wäßrige Lösung eine Pufferlösung ist.

4. Verfahren zur Konservierung von infektiösen rekombinanten Viren nach Anspruch 3, in dem die Pufferlösung ausgewählt ist aus dem Puffer Tris-HCI, Triethanolamin-, Diethanolamin-, Borat/HCl-, Glycin/NaOH-, EPPS- [N-(2-Hydroxyethyl)piperazin-N'-(3-propansulfonsäure)], Bicin-, TAPS- [N-Tris(hydroxymethyl)methyl-3-aminopropansulfonsäure] und Tricin-Lösungen, wobei der Puffer Tris-HCI bevorzugt ist.

5. Verfahren zur Konservierung von infektiösen rekombinanten Viren nach einem der Ansprüche 1 bis 4, in dem die wäßrige Lösung darüber hinaus mindestens ein Salz eines zweiwertigen Kations umfaßt, das ausgewählt ist aus MgCl₂, CaCl₂ und MnCl₂, wobei MgCl₂ bevorzugt ist.

6. Verfahren zur Konservierung von infektiösen rekombinanten Viren nach Anspruch 5, in dem das Salz des zweiwertigen Kations in der wäßrigen Lösung bei einer Konzentration von 0,1 bis 5 mM, vorzugsweise von 0,5 bis 2 mM und noch bevorzugter in der Größenordnung von 1 mM vorliegt.

7. Verfahren zur Konservierung von infektiösen rekombinanten Viren nach einem der Ansprüche 1 bis 6, in dem die wäßrige Lösung einen Puffer 10 mM Tris-HCl, 1 mM MgCl₂, 1 M Saccharose, pH 8,5 umfaßt.

8. Verfahren zur Konservierung von infektiösen rekombinanten Viren nach einem der Ansprüche 1 bis 6, in dem die wäßrige Lösung mindestens eine stabilisierende Verbindung umfaßt, die ausgewählt ist aus Salzen, vorzugsweise einwertigen, Aminosäuren und Tensiden.

9. Verfahren zur Konservierung von infektiösen rekombinanten Viren nach Anspruch 8, in dem das einwertige Salz ausgewählt ist aus NaCI und KCI, wobei NaCI bevorzugt ist.

10. Verfahren zur Konservierung von infektiösen rekombinanten Viren nach Anspruch 9, in dem das einwertige Salz in der wäßrigen Lösung bei einer Konzentration von 0,05 bis 1 M, vorzugsweise 0,1 bis 0,5 M, bevorzugter noch 0,1 bis 0,3 M vorliegt.

11. Verfahren zur Konservierung von infektiösen rekombinanten Viren nach Anspruch 8, in dem das Tensid Tween 80 oder Triton X-100 ist.

12. Verfahren zur Konservierung von infektiösen rekombinanten Viren nach Anspruch 11, in dem Tween 80 in der wäßrigen Lösung bei einer Konzentration von 0,001 bis 0,5 Gew.-%, vorzugsweise 0,002 bis 0,2 Gew.-% und noch bevorzugter in der Größenordnung von 0,005 Gew.-%, bezogen auf die Gesamt-Lösung, vorliegt.

13. Verfahren zur Konservierung von infektiösen rekombinanten Viren nach Anspruch 12, in dem die wäßrige Lösung einen Puffer 10 mM Tris-HCI, 1 mM MgCl₂, 150 mM NaCI, 0,05% Tween 80 und 1 M Saccharose, pH etwa 8,5, umfaßt.

14. Verfahren zur Konservierung von infektiösen rekombinanten Viren nach einem der Ansprüche 1 bis 13, in dem die Temperatur der Konservierung unter oder gleich + 4°C ist.

15. Verfahren zur Konservierung von infektiösen rekombinanten Viren nach einem der Ansprüche 1 bis 14, in dem die Viren in Lösung anschließend einer Lyophitisierung unterzogen werden.

16. Wäßrige Suspension von infektiösen rekombinanten Viren, die eine wäßrige Lösung von Saccharose bei einer Konzentration über 0,75 M, vorzugsweise von 0,75 M bis 1,5 M, bevorzugter noch gleich 1 M umfaßt, wobei die wäßrige Lösung einen pH von 8 bis 9 aufweist, vorzugsweise einen pH gleich 8,5.

17. Wäßrige Lösung von infektiösen rekombinanten Viren nach Anspruch 16, **dadurch gekennzeichnet, daß** die Viren Adenoviren oder Retroviren sind.

18. Wäßrige Suspension von infektiösen rekombinanten Viren nach einem der Ansprüche 16 und 17, in dem die wäßrige Lösung eine Pufferlösung ist.

19. Wäßrige Suspension von infektiösen rekombinanten Viren nach Anspruch 18, in dem die Pufferlösung ausgewählt ist aus dem Puffer Tris-HCI, Triethanolamin-, Diethanolamin-, Borat / HCI-, Glycin / NaOH-, EPPS- [N-(2-Hydroxyethyl)piperazin-N'-(3-propansulfonsäure)], Bicin-, TAPS- [N-Tris-(hydroxymethyl)methyl-3-aminopropansulfonsäure] und Tricin-Lösungen, wobei der Puffer Tris-HCl bevorzugt ist.

20. Wäßrige Suspension von infektiösen rekombinanten Viren nach einem der Ansprüche 16 bis 19, in der die wäßrige Lösung darüber hinaus mindestens ein Salz eines zweiwertigen Kations umfaßt, das ausgewählt ist aus MgCl₂, CaCl₂ und MnCl₂, wobei MgCl₂ bevorzugt ist.

21. Wäßrige Suspension von infektiösen rekombinanten Viren nach Anspruch 20, in der das Salz des zweiwertigen Kations in der wäßrigen Lösung bei einer Konzentration von 0,1 bis 5 mM, bevorzugt 0,5 bis 2 mM und noch bevorzugter in der Größenordnung von 1 mM vorliegt.

22. Wäßrige Suspension von infektiösen rekombinanten Viren nach einem der Ansprüche 16 bis 21, in der die wäßrige Lösung einen Puffer 10 mM Tris-HCI, 1 mM MgCl₂, 1 M Saccharose, pH 8,5, umfaßt.

23. Wäßrige Suspension von infektiösen rekombinanten Viren nach einem der Ansprüche 16 bis 21, in der die wäßrige Lösung mindestens eine stabilisierende Verbindung umfaßt, die ausgewählt ist aus Salzen, vorzugsweise einwertigen, Aminosäuren und Tensiden.

24. Wäßrige Suspension von infektiösen rekombinanten Viren nach Anspruch 23, in der das einwertige Salz ausgewählt ist aus NaCI und KCI, wobei NaCI bevorzugt ist.

25. Wäßrige Suspension von infektiösen rekombinanten Viren nach Anspruch 24, in der das einwertige Salz in der wäßrigen Lösung bei einer Konzentration von 0,05 bis 1 M, vorzugsweise 0,1 bis 0,5 M, noch bevorzugter von 0,1 bis 0,3 M vorliegt.

26. Wäßrige Suspension von infektiösen rekombinanten Viren nach Anspruch 23, in der das Tensid Tween 80 oder Triton X-100 ist.

27. Wäßrige Suspension von infektiösen rekombinanten Viren nach Anspruch 26, in der Tween 80 in der wäßrigen Lösung bei einer Konzentration von 0,001 bis 0,5 Gew.-%, vorzugsweise 0,002 bis 0,2 Gew.-% und noch bevorzugter in der Größenordnung von 0,005 Gew.-%, bezogen auf die Gesamt-Lösung, vorliegt.

28. Wäßrige Suspension von infektiösen rekombinanten Viren nach einem der Ansprüche 16 bis 27, die 10⁶ bis 10¹³ pfu/ml an infektiösen rekombinanten Viren umfaßt.

29. Wäßrige Lösung nach Anspruch 27, die einen Puffer 10 mM Tris-HCI, 1 mM MgCl₂, 150 mM NaCI, 0,05 % Tween 80 und 1 M Saccharose, pH etwa 8,5, umfaßt.

30. Pharmazeutische Zusammensetzung, umfassend eine wäßrige Suspension von infektiösen rekombinanten Viren nach einem der Ansprüche 16 bis 28 oder erhalten durch die Durchführung eines Verfahrens zur Konservierung von infektiösen rekombinanten Viren nach den Ansprüchen 1 bis 15 in Verbindung mit einem Träger, der unter pharmazeutischem Gesichtspunkt annehmbar ist.

31. Verwendung einer wäßrigen Suspension von infektiösen rekombinanten Viren nach einem der Ansprüche 16 bis 28 oder erhalten durch die Durchführung eines Verfahrens zur Konservierung von infektiösen rekombinanten Viren nach den Ansprüchen 1 bis 15 für die Herstellung eines Medikaments, das zur Behandlung oder Prophylaxe von genetischen Krankheiten, Krebsen, viralen Erkrankungen und Rückfallerkrankungen bestimmt ist.

32. Verwendung nach Anspruch 31 für die Herstellung eines Medikaments, das zur Behandlung oder Prophylaxe von Hämophilie, Mucoviszidose, Diabetes, Duchenne- und Becker-Myopathie bestimmt ist.

33. Verwendung nach Anspruch 31 für die Herstellung eines Medikaments, das zur Behandlung oder Prophylaxe von Hepatitiden oder AIDS bestimmt ist.

34. Verwendung nach Anspruch 31 für die Herstellung eines Medikaments, das zur Behandlung oder Prophylaxe von Infektionen bestimmt ist, die durch das Herpes-Virus oder das menschliche Papilloma-Virus hervorgerufen werden.

## Claims

1. Method of preserving infectious recombinant viruses in frozen or liquid form, in which the viruses are preserved in an aqueous solution comprising sucrose at a concentration of above 0.75 M, preferably between 0.75 M and 1.5 M, more preferably at a concentration of 1 M, the pH of the said aqueous solution being between 8 and 9, and preferably being 8.5.

2. Method of preserving infectious recombinant viruses according to Claim 1, in which the viruses are adenoviruses or retroviruses.

3. Method of preserving infectious recombinant viruses according to either of Claims 1 and 2, in which the aqueous solution is a buffer solution.

4. Method of preserving infectious recombinant viruses according to Claim 3, in which the buffer solution is selected from amongst Tris-HCl buffer and triethanolamine, diethanolamine, borate/HCl, glycine/NaOH, EPPS [N-(2-hydroxyethyl)piperazine-N'-(3-propanesulfonic) acid], bicine, TAPS [N-Tris-(hydroxymethyl)methyl-3-aminopropanesulfonic acid] and tricine solutions, Tris-HCl buffer being preferred.

5. Method of preserving infectious recombinant viruses according to any one of Claims 1 to 4, in which the aqueous solution additionally comprises at least one salt of a divalent cation selected from amongst MgCl₂, CaCl₂ and MnCl₂, MgCl₂ being preferred.

6. Method of preserving infectious recombinant viruses according to Claim 5, in which the salt of the divalent cation is present in the aqueous solution at a concentration of between 0.1 and 5 mM, preferably between 0.5 and 2 mM, more preferably in the order of 1 mM.

7. Method of preserving infectious recombinant viruses according to any one of Claims 1 to 6, in which the aqueous solution comprises 10 mM Tris-HCl buffer, 1 mM MgCl₂, 1 M sucrose, pH 8.5.

8. Method of preserving infectious recombinant viruses according to any one of Claims 1 to 6, in which the aqueous solution comprises at least one stabilizer selected from amongst salts, preferably monovalent salts, amino acids and surfactants.

9. Method of preserving infectious recombinant viruses according to Claim 8, in which the monovalent salt is selected from amongst NaCl and KCl, NaCl being preferred.

10. Method of preserving infectious recombinant viruses according to Claim 9, in which the monovalent salt is present in the aqueous solution at a concentration of between 0.05 and 1 M, preferably 0.1 and 0.5 M, more preferably between 0.1 and 0.3 M.

11. Method of preserving infectious recombinant viruses according to Claim 8, in which the surfactant is Tween 80 or Triton X-100.

12. Method of preserving infectious recombinant viruses according to Claim 11, in which Tween 80 is present in the aqueous solution at a concentration of between 0.001 and 0.5%, preferably between 0.002 and 0.2%, more preferably in the order of 0.005% by weight based on the total solution.

13. Method of preserving infectious recombinant viruses according to Claim 12, in which the aqueous solution comprises 10 mM Tris-HCl buffer, 1 mM MgCl₂, 150 mM NaCl, 0.05% of Tween 80 and 1 M sucrose, pH approx. 8.5.

14. Method of preserving infectious recombinant viruses according to any one of Claims 1 to 13, in which the preservation temperature is +4°C or less.

15. Method of preserving infectious recombinant viruses according to any one of Claims 1 to 14, in which the viruses in solution are then lyophilized.

16. Aqueous suspension of infectious recombinant viruses comprising an aqueous sucrose solution at a concentration of above 0.75 M, preferably between 0.75 M and 1.5 M, more preferably 1 M, the said aqueous solution having a pH of between 8 and 9, preferably a pH of 8.5.

17. Aqueous suspension of infectious recombinant viruses according to Claim 16, **characterized in that** the viruses are adenoviruses or retroviruses.

18. Aqueous suspension of infectious recombinant viruses according to any one of Claims 16 and 17, in which the aqueous solution is a buffer solution.

19. Aqueous suspension of infectious recombinant viruses according to Claim 18, in which the buffer solution is selected from amongst Tris-HCl buffer and triethanolamine, diethanolamine, borate/HCl, glycine/NaOH, EPPS [N-(2-hydroxyethyl)piperazine-N'-(3-propanesulfonic) acid], bicine, TAPS [N-Tris-(hydroxymethyl)methyl-3-aminopropanesulfonic acid] and tricine solutions, Tris-HCl buffer being preferred.

20. Aqueous suspension of infectious recombinant viruses according to any one of Claims 16 to 19, in which the aqueous solution additionally comprises at least one salt of a divalent cation selected from amongst MgCl₂, CaCl₂ and MnCl₂, MgCl₂ being preferred.

21. Aqueous suspension of infectious recombinant viruses according to Claim 20, in which the salt of the divalent cation is present in the aqueous solution at a concentration of between 0.1 and 5 mM, preferably between 0.5 and 2 mM, more preferably in the order of 1 mM.

22. Aqueous suspension of infectious recombinant viruses according to any one of Claims 16 to 21, in which the aqueous solution comprises 10 mM Tris-HCl buffer, 1 mM MgCl₂, 1 M sucrose, pH 8.5.

23. Aqueous suspension of infectious recombinant viruses according to any one of Claims 16 to 21, in which the aqueous solution comprises at least one stabilizer selected from amongst salts, preferably monovalent salts, amino acids and surfactants.

24. Aqueous suspension of infectious recombinant viruses according to Claim 23, in which the monovalent salt is selected from amongst NaCl and KCl, NaCl being preferred.

25. Aqueous suspension of infectious recombinant viruses according to Claim 24, in which the monovalent salt is present in the aqueous solution at a concentration of between 0.05 and 1 M, preferably 0.1 and 0.5 M, more preferably between 0.1 and 0.3 M.

26. Aqueous suspension of infectious recombinant viruses according to Claim 23, in which the surfactant is Tween 80 or Triton X-100.

27. Aqueous suspension of infectious recombinant viruses according to Claim 26, in which Tween 80 is present in the aqueous solution at a concentration of between 0.001 and 0.5%, preferably between 0.002 and 0.2%, more preferably in the order of 0.005% by weight based on the total solution.

28. Aqueous suspension of infectious recombinant viruses according to any one of Claims 16 to 27, comprising 10⁶ to 10¹³ pfu/ml infectious recombinant viruses.

29. Aqueous suspension according to Claim 27, comprising 10 mM Tris-HCl buffer, 1 mM MgCl₂, 150 mM NaCl, 0.05% of Tween 80 and 1 M sucrose, pH approx. 8.5.

30. Pharmaceutical composition comprising an aqueous suspension of infectious recombinant viruses according to any one of Claims 16 to 28 or obtained by making use of a method of preserving infectious recombinant viruses according to Claims 1 to 15, in association with a pharmaceutically acceptable vehicle.

31. Use of an aqueous suspension of infectious recombinant viruses according to any one of Claims 16 to 28 or obtained by making use of a method of preserving infectious recombinant viruses according to Claims 1 to 15, for the preparation of a medicament intended for the treatment or the prophylaxis of genetic diseases, cancers, viral diseases and recurrent diseases.

32. Use according to Claim 31 for the preparation of a medicament intended for the treatment or the prophylaxis of haemophilia, cystic fibrosis, diabetes, Duchenne's myopathy and Becker's myopathy.

33. Use according to Claim 31 for the preparation of a medicament intended for the treatment or the prophylaxis of hepatitis or of AIDS.

34. Use according to Claim 31 for the preparation of a medicament intended for the treatment or the prophylaxis of infections caused by herpes virus or human papilloma virus.
